(19) Europäisches Patentamt
European Patent Office
Office européen des brevets

(11) EP 1 490 692 B1

# (12) EUROPEAN PATENT SPECIFICATION

(45) Date of publication and mention of the grant of the patent:
**25.01.2006 Bulletin 2006/04**

(21) Application number: **03706511.7**

(22) Date of filing: **14.02.2003**

(51) Int Cl.:
*G01N 33/68* (2006.01) *C12Q 1/68* (2006.01)
*A01K 67/027* (2006.01) *A61K 38/00* (2006.01)
*A61K 48/00* (2006.01)

(86) International application number:
**PCT/EP2003/001493**

(87) International publication number:
**WO 2003/069347 (21.08.2003 Gazette 2003/34)**

## (54) DIAGNOSTIC AND THERAPEUTIC USE OF CAPS

DIAGNOSTISCHE UND THERAPEUTISCHE VERWENDUNG DES CAPS

UTILISATION DIAGNOSTIQUE ET THERAPEUTIQUE D'UNE PROTEINE ACTIVATRICE DE SECRETION VESICULAIRE DANS LES MALADIES NEURODEGENERATIVES

(84) Designated Contracting States:
**AT BE BG CH CY CZ DE DK EE ES FI FR GB GR HU IE IT LI LU MC NL PT SE SI SK TR**

(30) Priority: **14.02.2002 US 356155 P**
**14.02.2002 EP 02003431**

(43) Date of publication of application:
**29.12.2004 Bulletin 2004/53**

(73) Proprietor: **EVOTEC Neurosciences GmbH**
**22525 Hamburg (DE)**

(72) Inventors:
- **HIPFEL, Rainer**
  **D-69120 Heidelberg (DE)**
- **VON DER KAMMER, Heinz**
  **D-22607 Hamburg (DE)**
- **POHLNER, Johannes**
  **D-22175 Hamburg (DE)**

(74) Representative: **von Kreisler Selting Werner Patentanwälte**
**P.O. Box 10 22 41**
**50462 Köln (DE)**

(56) References cited:
**CA-A- 2 118 243** **US-A- 6 018 024**

- **LARA D R ET AL: "Increased serum S100B protein in schizophrenia: A study in medication-free patients" JOURNAL OF PSYCHIATRIC RESEARCH, vol. 35, no. 1, January 2001 (2001-01), pages 11-14, XP002263928 ISSN: 0022-3956**
- **HOYAUX DAPHNE ET AL: "S100A6, a calcium- and zinc-binding protein, is overexpressed in SOD1 mutant mice, a model for amyotrophic lateral sclerosis" BIOCHIMICA ET BIOPHYSICA ACTA, vol. 1498, no. 2-3, 20 December 2000 (2000-12-20), pages 264-272, XP002263929 ISSN: 0006-3002**
- **PALOTAS ANDRAS ET AL: "Comparative studies on (Ca2+)i-level of fibroblasts from Alzheimer patients and control individuals" NEUROCHEMICAL RESEARCH, vol. 26, no. 7, July 2001 (2001-07), pages 817-820, XP009022606 ISSN: 0364-3190**
- **LORING J F ET AL: "A GENE EXPRESSION PROFILE OF ALZHEIMER'S DISEASE" DNA AND CELL BIOLOGY, NEW YORK, NY, US, vol. 20, no. 11, November 2001 (2001-11), pages 683-695, XP001083747 ISSN: 1044-5498**
- **EFTHIMIOPOULOS S ET AL: "ENRICHMENT OF PRESENILIN 1 PEPTIDES IN NEURONAL LARGE DENSE-CORE AND SOMATODENDRITIC CLATHRIN-COATED VESICLES" JOURNAL OF NEUROCHEMISTRY, NEW YORK, NY, US, vol. 71, no. 6, December 1998 (1998-12), pages 2365-2372, XP001070239 ISSN: 0022-3042 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 490 692 B1

## Description

The present invention relates to methods of diagnosing, prognosticating and monitoring the progression of neurodegenerative diseases in a subject. Furthermore, methods of therapy control and screening for modulating agents of neurodegenerative diseases are provided. The invention also discloses pharmaceutical compositions, kits, and recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and it is probably the most devastating age-related neurodegenerative condition affecting about 10% of the population over 65 years of age and up to 45% over age 85 (for a recent review see Vickers et al., *Progress in Neurobiology* 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β (Aβ) protein evolves from the cleavage of the amyloid precursor protein (APP) by different kinds of proteases. The cleavage by the β/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, *Physiological Rev* 2001, 81: 741-66; Greenfield et al., *Frontiers Bioscience* 2000, 5: D72-83). Two types of plaques, diffuse plaques and neuritic plaques, can be detected in the brain of AD patients, the latter ones being the classical, most prevalent type. They are primarily found in the cerebral cortex and hippocampus. The neuritic plaques have a diameter of 50μm to 200μm and are composed of insoluble fibrillar amyloids, fragments of dead neurons, of microglia and astrocytes, and other components such as neurotransmitters, apolipoprotein E, glycosaminoglycans, α1-antichymotrypsin and others. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, *Acta Neuropathol* 1991, 82: 239-259). NFTs emerge inside neurons and consist of chemically altered tau, which forms paired helical filaments twisted around each other. Along the formation of NFTs, a loss of neurons can be observed. It is discussed that said neuron loss may be due to a damaged microtubule-associated transport system (Johnson and Jenkins, *J Alzheimers Dis* 1996, 1: 38-58; Johnson and Hartigan, *J Alzheimers Dis* 1999, 1: 329-351). The appearance of neurofibrillary tangles and their increasing number correlates well with the clinical severity of AD (Schmitt et al., *Neurology* 2000, 55: 370-376).

AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., *Annals of Neurology* 1981, 10: 184-92).

The age of onset of AD may vary within a range of 50 years, with early-onset AD occurring in people younger than 65 years of age, and late-onset of AD occurring in those older than 65 years. About 10% of all AD cases suffer from early-onset AD, with only 1-2% being familial, inherited cases.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., *Proc Natl Acad Sci USA* 1993, 90: 1977-81; Roses, *Ann NY Acad Sci* 1998, 855: 738-43). The polymorphic plasmaprotein ApoE plays a role in the intercellular cholesterol and phospholipid transport by binding low-density lipoprotein receptors, and it seems to play a role in neurite growth and regeneration. Studies linking the function of ApoE to AD pathology indicate that ApoE affects amyloid and tau metabolism. Thus, it is discussed to be an important factor for inhibiting axon outgrowth and for neurite and cell loss in AD. Efforts to detect further susceptibility genes and disease-linked polymorphisms, lead to the assumption that specific regions and genes on human chromosomes 10 and 12 may be associated with late-onset AD (Myers et al., *Science* 2000, 290: 2304-5; Bertram et al., *Science* 2000, 290: 2303; Scott et al., Am *J Hum Genet* 2000, 66: 922-32).

Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The mutations found to date account for only half of the familial AD cases, which is less than 2% of all AD patients. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

Synaptic transmission is a major mechanism for intercellular communication in the central and peripheral nervous system and neuroendocrine tissues. Many if not all nerve cells secrete bioactive peptides in addition to the conventional neurotransmitters. Two different types of secretory vesicles are involved, small synaptic vesicles and large, so-called dense-core vesicles (for review, Edwards, Curr. Biol. 1998, 8: R883-885). Small synaptic vesicles originate from the endosomal compartment and contain the classical neurotransmitters secreted and recycled at the synapse (e.g. acetylcholine, gamma-aminobutyric acid, glutamate). Large dense-core vesicles emerge from the trans-Golgi network and occur in the cell body and dendrites of a neuron as well as the axonal terminals to release their contents - upon $Ca^{2+}$ stimuli - at the presynaptic membrane. Large dense-core vesicles have proteins, peptides and biogenic amines as cargo that act as neuromodulators and hormones (e.g. neuropeptide Y in the human brain, Marx et al., Journal of Neuroscience 1999, 19: 8300-8311).

The calcium-dependent activator protein for secretion (CAPS) is a protein restricted in its expression to neuronal and neuroendocrine cells. The rat CAPS protein is a 2 x 145 kDa homodimeric protein originally identified as a cytosolic factor required for $Ca^{2+}$-triggered dense-core vesicle exocytosis in permeabilized PC12 adrenal neuroendocrine cells (Walent et al., Cell 1992, 70: 765-775). The rat PC12 cell is a widely used model system for mechanistic studies of vesicle secretion in neuroendocrine cells. A substantial fraction of CAPS was found to be peripherally membrane bound and stably associated with purified plasma membranes and dense-core vesicles but not with small synaptic vesicles prepared ex vivo from rat brain neuronal tissue (Berwin et al., Neuron 1998, 21: 137-145). Using isolated and permeabilized rat synaptosomes and neutralizing CAPS-directed antibodies, CAPS was shown to be essential for $Ca^{2+}$-induced exocytosis of norepinephrine-containing dense-core vesicles, but not glutamatergic synaptic vesicles (Martin and Kowalchyk, J. Biol. Chem. 1997, 272: 14447-14453; Tandon et al., Neuron 1998, 21: 147-154). CAPS from rat has binding sites for $Ca^{2+}$ and membrane phospholipids, in particular phosphatidylinositol-4,5-bisphosphate, and undergoes a conformational change upon binding of the phosphoinositide (Loyet et al., J. Biol. Chem. 1998, 273: 8337-8343). Mechanistically, CAPS has been proposed to act during a late step of $Ca^{2+}$-triggered exocytosis, i.e. after vesicle docking to the plasma membrane presynaptic termini and ATP-dependent priming of the vesicles but prior to or at the membrane fusion step of dense-core vesicles (Martin and Kowalchyk, J. Biol. Chem. 1997, 272: 14447-14453; Rupnik et al., Proc. Natl. Acad. Sci USA 2000, 97: 5627-5632).

Genetic studies with Caenorhabditis elegans and Drosophila melanogaster CAPS mutants confirmed the essential role for CAPS in synaptic transmission (Miller et al., Proc. Natl. Acad. Sci. USA 1996, 93: 12593-12598; Ailion et al., Proc. Natl. Acad. Sci. USA 1999, 96: 7394-7397; Renden et al., Neuron 2001, 31: 421-437). In a genetic screen for C. elegans Ric mutants (Ric = resistant to inhibitors of cholinesterase) the nematode CAPS ortholog Unc-31 was identified as a factor with a bona fide general role in the release of neurotransmitters (Miller et al., Proc. Natl. Acad. Sci. USA 1996, 93: 12593-12598). More recently, D. melanogaster dCAPS mutants were reported (Renden et al., Neuron 2001, 31: 421-437). The dCAPS null mutants die at the late embryo/early 1$^{st}$ instar larva junction indicating an essential role for dCAPS' function during hatching and postembryonic viability. Partial-loss-of function dCAPS mutants display defects in exocytosis of both small glutamatergic and dense-core vesicles. The glutamatergic phenotype in Drososphila dCAPS mutants and the cholinergic phenotype in C. elegans Unc-31 mutants probably are due to a loss of regulated biogenic amine or neuropeptide secretion from dense-core vesicles, which normally modulates synaptic vesicle exocytosis and facilitates synaptic transmission in a cell nonautonomous way (Renden et al., Neuron 2001, 31: 421-437). CAPS mutants of higher vertebrate model organisms have not been described yet.

Hirosawa and coworkers published a human CAPS ortholog in 1999 (Hirosawa et al., DNA Res 1999, 6: 329-336). The CAPS cDNA sequence named KIAA1121 was obtained from a human brain cDNA library and deposited in the GenBank (accession number AB032947). The gene locus was mapped to chromosome 3. The translated KIAA1121 polypeptide shows 98% amino acid sequence identity to the translated open reading frame of the full-length CAPS cDNA clone of rat (Ann et al., J. Biol. Chem 1997, 272: 19637-19640). In rat, a 5.6 kb CAPS mRNA was detected in brain, pancreas and adrenal gland but not in heart, placenta, lung, liver, skeletal muscle or kidney. Thus, the tissue distribution of CAPS in rat is consistent with the documented role of CAPS in exocytosis of secretory vesicles in rat neuronal and neuroendocrine cells.

To date no experiments have been described that show a relationship between a differential expression of the CAPS gene, a differential expression of a translation product of said gene and/or fragment of said translation product and/or level of activity of said translation product and/or fragment of said translation product and the pathology of neurodegenerative diseases. To date no mutations in the CAPS gene have been found to be associated with pathological phenotypes of said disorders. An indirect link between large dense-core vesicles and neurodegenerative diseases may be inferred from results showing an enrichment of presenitin-1 and proteolytic fragments thereof in large dense-core vesicles prepared from rat brain (Efthimiopoulos et al., J Neurochem 1998, 71: 2365-2372). Thus, we propose herein that a malfunctioning of CAPS may interfere with proper sorting of presenilin-1 and processing of APP in the neurodegenerative setting of Alzheimer's disease.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or acetylation, or lipidation, or by altered signal peptide cleavage or other types of maturation cleavage. These processes may occur post-translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in enzyme activity, a change in binding characteristics, or any other change or alteration in the biological, functional, or immunological properties of said translation product of a gene. The terms "agent", "reagent", or "compound" refer to any substance, chemical, composition or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared. The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of the protein of SEQ ID NO: 1. "Variants" of a protein molecule include, for example, proteins with conservative amino acid substitutions in highly conservative regions. "Proteins and polypeptides" of the present invention include variants, fragments and chemical derivatives of the protein comprising the amino acid sequences of SEQ ID NO. 1.

They can include proteins and polypeptides which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The term "isolated" as used herein is considered to refer to molecules that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state, and that such molecules can be produced by recombinant and/or synthetic means. Even if for said purposes those sequences may be introduced into living or non-living organisms by methods known to those skilled in the art, and even if those sequences are still present in said organisms, they are still considered to be isolated. In the present invention, the terms "risk", "susceptibility", and "predisposition" are tantamount and are used with respect to the probability of developing a neurodegenerative disease, preferably Alzheimer's disease. The term 'AD' shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, *Alzheimer's Disease and Related Disorders (Etiology, Pathogenesis and Therapeutics),* Wiley & Sons, New York, Weinheim, Toronto, 1999; Scinto and Daffner, *Early Diagnosis of Alzheirner's Disease,* Humana Press, Totowa, New Jersey, 2000; Mayeux and Christen, *Epidemiology of Alzheimer's Disease: From Gene to Prevention,* Springer Press, Berlin, Heidelberg, New York, 1999; Younkin, Tanzi and Christen, *Presenilins and Alzheimer's Disease,* Springer Press, Berlin, Heidelberg, New York, 1998).

Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, age-related macular degeneration, narcolepsy, motor neuron diseases, prion diseases, traumatic nerve injury and repair, and multiple sclerosis.

In one aspect, the invention features a method of diagnosing or prognosticating a neurodegenerative disease in a subject, or determining whether a subject is at increased risk of developing said disease. The method comprises: determining a level, or an activity, or both said level and said activity of (i) a transcription product of a gene coding for an activator protein for vesicle secretion, and/or of (ii) a translation product of a gene coding for an activator protein for vesicle secretion, and/or of (iii) a fragment, or derivative,or variant of said transcription or translation product in a sample from said subject and comparing said level, and/or said activity to a reference value representing a known disease or health status, thereby diagnosing or prognosticating said neurodegenerative disease in said subject, or determining whether said subject is at increased risk of developing said neurodegenerative disease.

The invention also relates to the construction and the use of primers and probes which are unique to the nucleic acid sequences, or fragments or variants thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for a neurodegenerative disease, in particular Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimers disease. This feature has utility for developing rapid DNA-based diagnostic tests, preferably also in the format of a kit.

In a further aspect, the invention features a method of monitoring the progression of a neurodegenerative disease in a subject. A level, or an activity, or both said level and said activity, of (i) a transcription product of a gene coding for an activator protein for vesicle secretion, and/or of (ii) a translation product of a gene coding for an activator protein for vesicle secretion, and/or of (iii) a fragment, or derivative, or variant of said transcription or translation product in a sample from said subject is determined. Said level and/or said activity is compared to a reference value representing a known disease or health status. Thereby the progression of said neurodegenerative disease in said subject is monitored.

In still a further aspect, the invention features a method of evaluating a treatment for a neurodegenerative disease, comprising determining a level, or an activity, or both said level and said activity of (i) a transcription product of a gene coding for an activator protein for vesicle secretion, and/or of (ii) a translation product of a gene coding for an activator protein for vesicle secretion, and/or of (iii) a fragment, or derivative, or variant of said transcription or translation product in a sample obtained from a subject being treated for said disease. Said level, or said activity, or both said level and said activity are compared to a reference value representing a known disease or health status, thereby evaluating the treatment for said neurodegenerative disease.

In a preferred embodiment of the herein claimed methods, kits, recombinant animals, molecules, assays, and uses of the instant invention, said gene coding for an activator protein for vesicle secretion is a member of the family of calcium-dependent activator proteins for secretion, in particular the calcium-dependent activator protein for secretion, herein termed CAPS. The present invention discloses the differential expression and regulation of the CAPS gene in specific brain regions of Alzheimer's disease patients. Consequently, the CAPS gene and its corresponding transcription and /or translation products may have a causative role in the regional selective neuronal degeneration typically observed in Alzheimer's disease. Alternatively, CAPS may confer a neuroprotective function to the remaining surviving nerve cells. Based on these disclosures, the present invention has utility for the diagnostic evaluation and prognosis as well as for the identification of a predisposition to a neurodegenerative disease, in particular Alzheimer's disease. Furthermore, the present invention provides methods for the diagnostic monitoring of patients undergoing treatment for such a disease.

In a further preferred embodiment of the herein claimed methods, kits, recombinant animals, molecules, assays, and uses of the instant invention, said neurodegenerative disease or disorder is Alzheimer's disease, and said subjects suffer from Alzheimer's disease.

It is preferred that the sample to be analyzed and determined is selected from the group comprising brain tissue or other body cells. The sample can also comprise cerebrospinal fluid or other body fluids including saliva, urine, serum plasma, or mucus. Preferably, the methods of diagnosis, prognosis, monitoring the progression or evaluating a treatment for a neurodegenerative disease, according to the instant invention, can be practiced *ex corpore,* and such methods preferably relate to samples, for instance, body fluids or cells, removed, collected, or isolated from a subject or patient.

In further preferred embodiments, said reference value is that of a level, or an activity, or both said level and said activity of an activator protein for vesicle secretion (i) a transcription product of a gene coding for an activator protein for vesicle secretion, and/or of (ii) a translation product of a gene coding for an activator protein for vesicle secretion, and/or of (iii) a fragment, or derivative, or variant of said transcription or translation product in a sample from a subject not suffering from said neurodegenerative disease.

In preferred embodiments, an alteration, i.e. an increase or decrease, in the level and/or activity of a transcription product of the gene coding for CAPS, i.e. CAPS mRNA, and/or of a translation product of the gene coding for CAPS, i.e. CAPS protein in a sample cell, or tissue, or body fluid from said subject relative to a reference value representing a known health status indicates a diagnosis, or prognosis, or increased risk of becoming diseased with a neurodegenerative disease, particularly Alzheimer's disease.

In preferred embodiments, measurement of the level of transcription products of a gene coding for an activator protein for vesicle secretion is performed in a sample from a subject using a quantitative PCR-analysis with primer combinations to amplify said gene specific sequences from cDNA obtained by reverse transcription of RNA extracted from a sample of a subject. A Northern blot with probes specific for said gene can also be applied. It might also be preferred to measure transcription products by means of chip-based microarray technologies. These techniques are known to those of ordinary skill in the art (see Sambrook and Russell, *Molecular Cloning: A Laboratory Manual,* Cold Spring Harbor Laboratory Press, Cold Spring Harbor, New York, 2001; Schena M., *Microarray Biochip Technology,* Eaton Publishing, Natick, MA, 2000). An example of an immunoassay is the detection and measurement of enzyme activity as disclosed and described in the patent application WO 02/14543.

Furthermore, the level, or the activity, or both said level and said activity of a translation product of a gene coding for an activator protein for vesicle secretion and/or the level, or the activity of a fragment, or derivative, or variant of said translation product, can be detected using an immunoassay, an activity assay and/or binding assay. These assays can measure the amount of binding between said protein molecule and an anti-protein antibody by the use, for instance, of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, *Using Antibodies: A Laboratory Manual,* Cold Spring Harbor Laboratory Press, Cold Spring Harbor, New York, 1999 and Edwards R, *Immunodiagnostics: A Practical Approach,* Oxford University Press, Oxford; England, 1999). All these detection techniques may also be employed in the format of microarrays, protein-arrays, antibody microarrays, tissue microarrays, electronic biochip or protein-chip based technologies (see Schena M., *Microarray Biochip Technology*, Eaton Publishing, Natick, MA, 2000).

In a preferred embodiment, the level, or the activity, or both said level and said activity of (i) a transcription product of a gene coding for an activator protein for vesicle secretion, and/or of (ii) a translation product of a gene coding for an activator protein for vesicle secretion, and/or of (iii) a fragment, or derivative, or variant of said transcription or translation product in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In another aspect, the invention features a kit for diagnosing or prognosticating a neurodegenerative disease, in particular Alzheimer's disease, in a subject, or determining the propensity or predisposition of a subject to develop a neurodegenerative disease, in particular Alzheimer's disease, said kit comprising:

(a) at least one reagent which is selected from the group consisting of (i) reagents that selectively detect a transcription product of a gene coding for an activator protein for vesicle secretion (ii) reagents that selectively detect a translation product of a gene coding for an activator protein for vesicle secretion; and
(b) instruction for diagnosing, or prognosticating a neurodegenerative disease, in particular Alzheimer's disease, or determining the propensity or predisposition of a subject to develop such a disease by

- detecting a level, or an activity, or both said level and said activity, of said transcription product and/or said translation product of a gene coding for an activator protein for vesicle secretion, in a sample from said subject; and
- diagnosing or prognosticating a neurodegenerative disease, in particular Alzheimer's disease, or determining the propensity or predisposition of said subject to develop such a disease,

wherein a varied level, or activity, or both said level and said activity, of said transcription product and/or said translation product compared to a reference value representing a known health status; or a level, or activity, or both said level and said activity, of said transcription product and/or said translation product similar or equal to a reference value representing a known disease status, indicates a diagnosis or prognosis of a neurodegenerative disease, in particular Alzheimer's disease, or an increased propensity or predisposition of developing such a disease. The kit, according to the present invention, may be particularly useful for the identification of individuals that are at risk of developing a neurodegenerative disease, in particular Alzheimer's disease. Consequently, the kit, according to the invention, may serve as a means for targeting identified individuals for early preventive measures or therapeutic intervention prior to disease onset, before irreversible damage in the course of the disease has been inflicted. Furthermore, in preferred embodiments, the kit featured in the invention is useful for monitoring a progression of a neurodegenerative disease, in particular Alzheimer's disease, in a subject, as well as monitoring success or failure of therapeutic treatment for such a disease of said subject.

In one aspect, the present invention also provides a kit comprising one or more containers filled with a therapeutically or prophylactically effective amount of said pharmaceutical composition.

In a further aspect, the invention features a recombinant, non-human animal comprising a non-native gene sequence coding for an activator protein for vesicle secretion, or a fragment, or a derivative, or a variant thereof. The generation of said recombinant, non-human animal comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said endogenous gene, wherein said gene is mis-expressed, or under-expressed, or over-expressed, and wherein said disruption or alteration results in said non-human animal exhibiting a predisposition to developing symptoms of neuropathology similar to a neurodegenerative disease, in particular Alzheimer's disease. Strategies and techniques for the generation and construction of such an animal are known to those of ordinary skill in the art (see e.g. Capecchi, *Science* 1989, 244: 1288-1292 and Hogan et al., 1994, *Manipulating the Mouse Embryo: A Laboratory Manual,* Cold Spring Harbor Laboratory Press, Cold Spring Harbor, New York and Jackson and Abbott, *Mouse Genetics and Transgenics: A Practical Approach,* Oxford University Press, Oxford, England, 1999). It is preferred to make use of such a recombinant non-human animal as an animal model for investigating neurodegenerative diseases, in particular Alzheimer's disease. Such an animal may be useful for screening, testing and validating compounds, agents and modulators in the development of diagnostics and therapeutics to treat neurodegenerative diseases, in particular Alzheimer's disease. In preferred embodiments, said recombinant, non-human animal comprises a non-native gene sequence coding for an activator protein for vesicle secretion, in particular CAPS, or a fragment, or derivative, or variant thereof.

In another aspect, the invention features an assay for screening for a modulator of neurodegenerative diseases, in particular Alzheimer's disease, or related diseases and disorders of one or more substances selected from the group consisting of (i) a gene coding for an activator protein for vesicle secretion, and/or (ii) a transcription product of a gene coding for an activator protein for vesicle secretion, and/or (iii) a translation product of a gene coding for an activator protein for vesicle secretion, and/or (iv) a fragment, or derivative, or variant of (i) to (iii). This screening method comprises (a) contacting a cell with a test compound, and (b) measuring the activity, or the level, or both the activity and the level of one or more substances recited in (i) to (iv), and (c) measuring the activity, or the level, or both the activity and the level of said substances in a control cell not contacted with said test compound, and (d) comparing the levels of the substance in the cells of step (b) and (c), wherein an alteration in the activity and/or level of said substances in the contacted cells indicates that the test compound is a modulator of said diseases and disorders.

In one further aspect, the invention features a screening assay for a modulator of neurodegenerative diseases, in particular Alzheimer's disease, or related diseases and disorders of one or more substances selected from the group consisting of (i) a gene coding for an activator protein for vesicle secretion, and/or (ii) a transcription product of a gene coding for an activator protein for vesicle secretion, and/or (iii) a translation product of a gene coding for an activator protein for vesicle secretion, and/or (iv) a fragment, or derivative, or variant of (i) to (iii), comprising (a) administering a test compound to a test animal which is predisposed to developing or has already developed symptoms of a neurodegenerative disease or related diseases or disorders, and (b) measuring the activity and/or level of one or more substances recited in (i) to (iv), and (c) measuring the activity and/or level of said substances in a matched control animal which is equally predisposed to developing or has already developed said symptoms and to which animal no such test compound has been administered, and (d) comparing the activity and/or level of the substance in the animals of step (b) and (c), wherein an alteration in the activity and/or level of substances in the test animal indicates that the test compound is a modulator of said diseases and disorders.

In a preferred embodiment, said test animal and/or said control animal is a recombinant, non-human animal which expresses an activator protein for vesicle secretion, or a fragment thereof, or a derivative thereof, under the control of a transcriptional regulatory element which is not the native activator protein for.vesicle secretion transcriptional control regulatory element.

In another embodiment, the present invention provides a method for producing a medicament comprising the steps of (i) identifying a modulator of neurodegenerative diseases by a method of the aforementioned screening assays and (ii) admixing the modulator with a pharmaceutical carrier. However, said modulator may also be identifiable by other types of screening assays.

In another aspect, the present invention provides for an assay for testing a compound, preferably for screening a plurality of compounds, for inhibition of binding between a ligand and an activator protein for vesicle secretion, or a fragment or derivative thereof. Said screening assay comprises the steps of (i) adding a liquid suspension of said activator protein for vesicle secretion, or a fragment, or derivative, or variant thereof, to a plurality of containers, and (ii) adding a compound or a plurality of compounds to be screened for said inhibition to said plurality of containers, and (iii) adding fluorescently labelled ligand to said containers, and (iv) incubating said activator protein for vesicle secretion, or said fragment, or derivative, or variant thereof, and said compound or plurality of compounds, and said fluorescently labelled ligand, and (v) measuring the amounts of fluorescence associated with said activator protein for vesicle secretion, or with said fragment, or derivative, or variant thereof, and (vi) determining the degree of inhibition by one or more of said compounds of binding of said ligand to said activator protein for vesicle secretion, or said fragment, or derivative, or variant thereof. Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of a gene coding for an activator protein for vesicle secretion, or a fragment, or derivative, or variant thereof. One example of a fluorescent binding assay, in this case based on the use of carrier particles, is disclosed and described in patent application WO 00/52451. A further example is the competitive assay method as described in patent WO 02/01226. Preferred signal detection methods for screening assays of the instant invention are described in the following patent applications: WO 96/13744, WO 98/16814, WO 98/23942, WO 99/17086, WO 99/34195, WO 00/66985, WO 01/59436, WO 01/59416.

In one further embodiment, the present invention provides a method for producing a medicament comprising the steps of (i) identifying a compound as an inhibitor of binding between a ligand and a gene product of a gene coding for an activator protein for vesicle secretion by the aforementioned inhibitory binding assay and (ii) admixing the compound with a pharmaceutical carrier. However, said compound may also be identifiable by other types of screening assays.

In another aspect, the invention features an assay for testing a compound, preferably for screening a plurality of compounds to determine the degree of binding of said compounds to an activator protein for vesicle secretion, or to a fragment, or derivative, or variant thereof. Said screening assay comprises (i) adding a liquid suspension of said activator protein for vesicle secretion, or a fragment, or derivative, or variant thereof, to a plurality of containers, and (ii) adding a fluorescently labelled compound or a plurality of fluorescently labelled compounds to be screened for said binding to said plurality of containers, and (iii) incubating said activator protein for vesicle secretion, or said fragment, or derivative, or variant thereof, and said fluorescently labelled compound or fluorescently labelled compounds, and (iv) measuring the amounts of fluorescence associated with said activator protein for vesicle secretion, or with said fragment, or derivative, or variant thereof, and (v) determining the degree of binding by one or more of said compounds to said activator protein for vesicle secretion, or said fragment, or derivative, or variant thereof. In this type of assay it might be preferred to use a fluorescent label. However, any other type of detectable label might also be employed. Said method may be useful for the identification of novel compounds as well as for evaluating compounds which have been improved or otherwise optimized in their ability to bind to an activator protein for vesicle secretion gene product, or fragment, or derivative, or variant thereof.

In one further embodiment, the present invention provides a method for producing a medicament comprising the steps of (i) identifying a compound as a binder to a gene product of a gene coding for an activator protein for vesicle secretion by the aforementioned binding assays and (ii) admixing the compound with a pharmaceutical carrier. However, said compound may also be identifiable by other types of screening assays.

In another embodiment, the present invention provides for a medicament obtainable by any of the methods according to the herein claimed screening assays. In one further embodiment, the instant invention provides for a medicament obtained by any of the methods according to the herein claimed screening assays.

The present invention features a protein molecule shown in SEQ ID NO: 1, or a fragment, or derivative, or variant thereof, for use as a diagnostic target for detecting a neurodegenerative disease, preferably Alzheimer's disease.

The present invention further features a protein molecule shown in SEQ ID NO: 1, or a fragment, or derivative, or variant thereof, for use as a screening target for reagents or compounds preventing, or treating, or ameliorating a neurodegenerative disease, preferably Alzheimer's disease.

In all types of assays disclosed herein it is preferred to study a member of the activator protein for vesicle secretion gene family. It is particularly preferred to conduct screening assays with the activator protein for vesicle secretion CAPS.

The present invention features an antibody which is specifically immunoreactive with an immunogen, wherein said immunogen is a translation product of the CAPS gene or a fragment, or derivative, or variant thereof. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., *Antibodies, A Laboratory Manual,* Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, *Recombinant Antibodies*, Wiley-Liss, New York, NY, 1999). Antibodies of the present invention are useful, for instance, in a variety of diagnostic and therapeutic methods, based on state-in-the-art techniques (see Harlow and Lane, *Using Antibodies: A Laboratory Manual,* Cold Spring Harbor Laboratory Press, Cold Spring Harbor, New York, 1999 and Edwards R., *Immunodiagnostics: A Practical Approach,* Oxford University Press, Oxford, England, 1999) such as enzyme-immuno assays (e.g. enzyme-linked immunosorbent assay, ELISA), radioimmuno assays, chemoluminescence-immuno assays, Western-blot, immunoprecipitation and antibody microarrays. These methods involve the detection of translation products of the CAPS gene.

In a preferred embodiment of the present invention, said antibodies can be used for detecting the pathological state of a cell in a sample from a subject, comprising immunocytochemical staining of said cell with said antibody, wherein an altered degree of staining, or an altered staining pattern in said cell compared to a cell representing a known health status indicates a pathological state of said cell. Preferably, the pathological state relates to a neurodegenerative disease, in particular to Alzheimer's disease. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples, which are illustrative only, and not intended to limit the remainder of the disclosure in any way.

Figure 1 depicts the brain regions with selective vulnerability to neuronal loss and degeneration in Alzheimer's disease. Primarily, neurons within the inferior temporal lobe, the entorhinal cortex, the hippocampus, and the amygdala are subject to degenerative processes in Alzheimer's disease (Terry et al., *Annals of Neurology* 1981, 10:184-192). These brain regions are mostly involved in the processing of learning and memory functions. In contrast, neurons within the frontal cortex, the occipital cortex, and the cerebellum remain largely intact and preserved from neurodegenerative processes in Alzheimer's disease. Brain tissues from the frontal cortex (F) and the temporal cortex (T) of Alzheimer's disease patients and healthy, age-matched control individuals were used for the herein disclosed examples. For illustrative purposes, the image of a normal healthy brain was taken from a publication by Strange (*Brain Biochemistry and Brain Disorders,* Oxford University Press, Oxford, 1992, p.4).

Figure 2 discloses the initial identification of the differential expression of CAPS in a suppressive subtractive hybridization screen. The figure shows a clipping of a large-scale dot blot hybridization experiment. Individual cDNA clones from a temporally subtracted library were arrayed onto a nylon membrane and hybridized with probes enriched for genes expressed in the frontal cortex (F) and the temporal cortex (T) of an Alzheimer's disease patient, Ia) clone T20-B06; Ib) clone T20-C06; Ic) clone T20-D06; CAPS ; IIa) clone T20-B07; IIb) clone T20-C07; IIc) clone T20-D07. Note the significantly increased intensity of the hybridization signal for CAPS in panel F as compared to the signal in panel T (see arrowheads).

Figure 3 illustrates the verification of the differential expression of the CAPS gene in AD brain tissues by quantitative RT-PCR analysis. Quantification of RT-PCR products from RNA samples collected from the frontal cortex (F) in comparison to the temporal cortex (T) is shown in Figure 3. The quantification of samples from Alzheimer's disease patients (Figure 3a) and of healthy, age-matched control individuals (Figure 3b) was performed by the LightCycler rapid thermal cycling technique. The data were normalized to the combined average values of a set of standard genes which showed no significant differences in their gene expression levels. Said set of standard genes consisted of genes for the ribosomal protein S9, cyclophilin B, the transferrin receptor, GAPDH, and beta-actin. The figure depicts the kinetics of amplification by plotting the cycle number against the amount of amplified material as measured by its fluorescence. Note that the amplification kinetics of CAPS cDNA from both the frontal and temporal cortices of a normal control individual during the exponential phase of the reaction are juxtaposed (Figure 3b, arrowheads), whereas in Alzheimer's disease (Figure 3a, arrowheads) there is a significant separation of the corresponding curves, indicating a differential expression of the CAPS gene in the respective analyzed brain regions.

Figure 4 discloses SEQ ID NO: 1, the amino acid sequence of the CAPS protein (GenBank accession number Q9ULU8). The full-length human CAPS protein comprises 1353 amino acids.

Figure 5 depicts SEQ ID NO: 2, the nucleotide sequence of the 641 bp CAPS cDNA fragment, identified and obtained by suppressive subtractive hybridization cloning (sequence in 5' to 3' direction).

Figure 6 outlines the sequence alignment of SEQ ID NO: 2, the 641 bp CAPS cDNA fragment, with the nucleotide sequence of the calcium-dependent activator protein for secretion KIAA1121 (GenBank accession number AB032947).

Figure 7 charts the schematic alignment of SEQ ID NO: 2, the CAPS cDNA fragment, to the nucleotide sequence of the calcium-dependent activator protein for secretion KIAA1121 (GenBank accession number AB032947). The open box represents the KIAA1121 open reading frame, thin bars represent the 5' and 3' untranslated regions (UTR) of the mRNA, respectively. The CAPS cDNA fragment is identical, albeit alternatively spliced, to two parts of the 4062 bp KIAA1121 coding sequence.

Figure 8 depicts human cerebral cortex labeled with anti-CAPS mouse monoclonal antibodies (green signal). Immunoreactivity of the activator protein for vesicle secretion CAPS was detected in the pre-central cortex (CT) but not in the white matter (WM) (Figure 9a, low magnification). The cortex showed a punctate immunostaining pattern in neuronal peripheries and neurites (Figure 9b, high magnification), suggesting a neuronal membrane-association of CAPS. This staining pattern may be indicative of large dense-core vesicle clusters. Blue signals indicate nuclei stained with DAPI.

Table 1 lists the expression levels in the frontal cortex relative to the temporal cortex for the CAPS gene in seven Alzheimer's disease patients, herein identified by internal reference numbers P010, P011, P012, P014, P016, P017, P019 (0.90 to 4.73 fold) and five healthy, age-matched control individuals, herein identified by internal reference numbers C005, C008, C011, C012, C014 (0.52 to 1.18 fold). The values shown are reciprocal values according to the formula described herein (see below).

EXAMPLE I:

(i) <u>Brain tissue dissection from patients with Alzheimer's disease:</u>

Brain tissues from Alzheimer's disease patients and age-matched control-subjects were collected within 6 hours post-mortem and immediately frozen on dry ice. Sample sections from each tissue were fixed in paraformaldehyde for histopathological confirmation of the diagnosis. Brain areas for differential expression analysis were identified (see Figure 1) and stored at - 80 °C until RNA extractions were performed.

(ii) Isolation of total RNA:

Total RNA was extracted from post-mortem brain tissue by using the RNeasy kit (Qiagen) according to the manufacturer's protocol. The accurate RNA concentration and the RNA quality was determined with the DNA LabChip system using the Agilent 2100 Bioanalyzer (Agilent Technologies).
For additional quality testing of the prepared RNA, i.e. exclusion of partial degradation and testing for DNA contamination, specifically designed intronic GAPDH oligonucleotides and genomic DNA as reference control were used to generate a melting curve with the LightCycler technology as described in the corresponding protocol (Roche).

(iii) cDNA synthesis and identification of differentially expressed genes by suppressive subtractive hybridization:

This technique compares two populations of RNA and provides clones of genes that are expressed in one population but not in the other. The applied technique was described in detail by Diatchenko et al. (Proc. Natl. Acad. Sci. USA 1996, 93: 6025-30). In the present invention, RNA populations from post-mortem brain tissues from Alzheimer's disease patients were compared. Specifically, RNA of the inferior frontal cortex was subtracted from RNA of the inferior temporal cortex. The necessary reagents were taken from the PCR-Select cDNA subtraction kit (Clontech), and all steps were performed as described in the manufacturer's protocol. Specifically, 2μg RNA each were used for first-strand and second-strand cDNA synthesis. After RsaI-digestion and adaptor ligation hybridization of tester and driver was performed for 8 hours (first hybridization) and 15 hours (second hybridization) at 68 °C. Two PCR steps were performed to amplify differentially expressed genes (first PCR: 27 cycles of 94 °C and 30 sec, 66 °C and 30 sec, and 72 °C and 1.5 min; nested PCR: 12 cycles of 94 °C and 30 sec, 66 °C and 30 sec, and 72 °C and 1.5 min) using adaptor specific primers (included in the subtraction kit) and 50x Advantage Polymerase Mix (Clontech). Efficiencies of RsaI-digestions, adaptor ligations and subtractive hybridizations were checked as recommended in the kit. Subtracted cDNAs were inserted into the pCR vector and transformed into *E.coli* INVαF' cells (Invitrogen).
To isolate individual cDNAs of the subtracted library, single bacterial transformants were incubated in 100 μl LB (with 50 μg/ml ampicillin) at 37 °C for at least 4 hours. Inserts were PCR amplified (95 °C and 30 sec, 68 °C and 3 min for 30 cycles) in a volume of 20 μl containing 10 mM Tris-HCl pH 9.0, 1.5 mM $MgCl_2$, 50 mM KCl, 200 μM dNTP, 0.5 μM adaptor specific primers (included in the subtraction kit), 1.5 Units Taq polymerase (Pharmacia Biotech), and 1 μl of bacterial culture.
1.5 μl of a mixture containing 3 μl PCR amplified inserts and 2 μl 0.3 N NaOH/15 % Ficoll were spotted onto a positively charged nylon membrane (Roche). In this way, hundreds of spots were arrayed on duplicate filters for subsequent hybridization analysis. The differential screening step consisted of hybridizations of the subtracted library with itself to minimize background (Wang and Brown, Proc. Natl. Acad. Sci. USA 1991, 88: 11505-9). The probes were generated from the nested PCR product of the subtraction following the instructions of the Clontech subtraction kit. Labeling with digoxigenin was performed with the DIG DNA Labeling Kit (Roche). Hybridizations were carried out overnight in DIG Easy HYB (Roche) at 43 °C. The filters were washed twice in 2 x SSC / 0.5 % SDS at 68 °C for 15 min and twice in 0.1 x SSC / 0.5 % SDS at 68 °C for 15 min, and subjected to detection using anti-DIG-AP conjugates and CDP-Star as chemiluminescent substrate according to the instructions of the DIG DNA Detection Kit (Roche). Blots were exposed to Kodak Biomax MR chemiluminescent film at room temperature for several minutes. The nucleotide sequences of clones of interest were obtained using methods well known to those skilled in the art. For nucleotide sequence analyses and homology searches, computer algorithms of the University of Wisconsin Genetics Computer Group (GCG) in conjunction with publicly available nucleotide and peptide sequence information (GenBank and EMBL databases) were employed. The results of one such subtractive hybridization experiment for the CAPS gene are shown in Figure 2.

(iv) Confirmation of differential expression by quantitative RT-PCR:

Positive corroboration of differential expression of the CAPS gene was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than an endpoint readout. The ratio of CAPS cDNA from the temporal cortex and frontal cortex was determined (relative quantification). First, a standard curve was generated to determine the efficiency of the PCR with specific primers for CAPS:

5'- GGCGTGTCCCTGTTATCAGC -3'

and

5'- TGCCCTCACATCCATTTACCA -3'.

PCR amplification (95 °C and 1 sec, 56 °C and 5 sec, and 72 °C and 5 sec) was performed in a volume of 20 μl containing Lightcycler-FastStart DNA Master SYBR Green I -ready-to-use mix (contains FastStart Taq DNA polymerase, reaction buffer, dNTP mix with dUTP instead of dTTP, SYBR Green I dye, and 1 mM $MgCl_2$, Roche), 0,5 μM primers, 2 μl of a cDNA dilution series (final concentration of 40, 20, 10, 5, 1 and 0,5 ng human total brain cDNA, Clontech) and depending on the primers used, additional 3 mM $MgCl_2$. Melting curve analysis revealed a single peak at approximately 80.5°C with no visible primer dimers. Quality and size of the PCR product was determined with the DNA LabChip system (Agilent 2100 Bioanalyzer, Agilent Technologies). A single peak at the expected size of 83 bp for the CAPS gene was observed in the electropherogram of the sample.

In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTT-TGCC-3' except for $MgCl_2$ (an additional 1 mM was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'-TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'- TGGAACGGTGAAGGTGACA-3' and 5'- GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers 5'-CGTCATGGGTGTGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'- GTCGCTGGTCAGTTCGTGATT-3' and 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for CAPS and the five reference standard genes. The slopes and the intercepts of the standard curves (i.e. linear regressions) were calculated for all genes.

In a second step, cDNA from temporal cortex and frontal cortex was analyzed in parallel and normalized to cyctophilin B. The $C_t$ values were measured and converted to ng total brain cDNA using the corresponding standard curves:

$$10 \wedge ( (C_t \text{ value - intercept}) / \text{slope} ) \qquad [\text{ng total brain cDNA}]$$

The values of temporal and frontal cortex CAPS cDNAs were normalized to cyclophilin B, and the ratio was calculated using the following formula:

$$\text{Ratio} = \frac{\text{CAPS temporal [ng] / cyclophilin B temporal [ng]}}{\text{CAPS frontal [ng] / cyclophilin B frontal [ng]}}$$

In a third step, the set of reference standard genes was analyzed in parallel to determine the mean average value of the temporal to frontal ratios of expression levels of the reference standard genes for each individual brain sample. As cyclophilin B was analyzed in step 2 and step 3, and the ratio from one gene to another gene remained constant in different runs, it was possible to normalize CAPS to the mean average value of the set of reference standard genes instead of normalizing to one single gene alone. The calculation was performed by dividing the ratio shown above by the deviation of cyclophilin B from the mean value of all housekeeping genes.

The results of one such quantitative RT-PCR analysis for the CAPS gene are shown in Figure 3.

(v) Immunohistochemistry:

For immunofluorescence staining of the activator protein for vesicle secretion CAPS in human brain, frozen sections were prepared from post-mortem pre-central gyrus of a donor person (Cryostat Leica CM3050S) and fixed in acetone for 10 min. After washing in PBS, the sections were pre-incubated with blocking buffer (10% normal goat serum, 0.2% Triton X-100 in PBS) for 30min, and then incubated with anti-CAPS mouse monoclonal antibodies (1:10 dilution in blocking buffer, BD Biosciences, Heidelberg) overnight at 4°C. After rinsing three times in 0.1% Triton X-100/PBS, the sections were incubated with FITC-conjugated goat anti-mouse IgG (1:150 diluted in 1% BSA/PBS) for 2 hours at room temperature, and then again washed in PBS. Staining of the nuclei was performed by incubation of the sections with 5μM DAPI in PBS for 3 min (blue signal). In order to block the autofluoresence of lipofuscin in human brain, the sections were treated with 1% Sudan Black B in 70% ethanol for 2-10 min at room temperature, sequentially dipped in 70% ethanol, destilled water and PBS. The sections were coverslipped by 'Vectrashield mounting medium' (Vector Laboratories, Burlingame, CA) and observed under an inverted microscope (IX81, Olympus Optical). The digtal images were captured with the appropriate software (AnalySiS, Olympus Optical).

## Claims

**1.** A method of diagnosing or prognostication a neurodegenerative disease in a subject, or determining whether a subject is at increased risk of developing said disease, comprising:

determining a level and/or an activity of

(i) a transcription product of a gene coding for the activator protein for vesicle secretion shown in SEQ ID NO: 1, and/or
(ii) a translation product of a gene coding for the activator protein for vesicle secretion shown in SEQ ID NO: 1, and/or
(iii) a fragment, or derivative, or variant of said transcription or translation product,

in a sample obtained from said subject and comparing said level and/or said activity to a reference value representing a known disease or health status, thereby diagnosing or prognosticating said neurodegenerative disease in said subject, or determining whether said subject is at increased risk of developing said neurodegenerative disease.

**2.** A method of monitoring the progression of a neurodegenerative disease in a subject, comprising:

determining a level and/or an activity of

(i) a transcription product of a gene coding for the activator protein for vesicle secretion shown in SEQ ID NO: 1, and/or
(ii) a translation product of a gene coding for the activator protein for vesicle secretion shown in SEQ ID NO: 1, and/or
(iii) a fragment, or derivative, or variant of said transcription or translation product,

in a sample obtained from said subject and comparing said level and/or said activity to a reference value representing a known disease or health status, thereby monitoring the progression of said neurodegenerative disease in said subject.

**3.** A method of evaluating a treatment for a neurodegenerative disease, comprising:

determining a level and/or an activity of

(i) a transcription product of a gene coding for the activator protein for vesicle secretion shown in SEQ ID NO: 1, and/or
(ii) a translation product of a gene coding for the activator protein for vesicle secretion shown in SEQ ID NO: 1, and/or
(iii) a fragment, or derivative, or variant of said transcription or translation product,

in a sample obtained from a subject being treated for said disease and comparing said level and/or said activity to a reference value representing a known disease or health status, thereby evaluating said treatment for said neurodegenerative disease.

**4.** The method according to any of claims 1 to 3 wherein said neurodegenerative disease is Alzheimer's disease.

**5.** The method according to any of claims 1 to 4 wherein said sample comprises a cell, or a tissue, or an organ, or a body fluid, in particular cerebrospinal fluid or blood.

**6.** The method according to any of claims 1 to 5 wherein said reference value is that of a level and/or an activity of

(i) a transcription product of a gene coding for the activator protein for vesicle secretion shown in SEQ ID NO: 1, and/or
(ii) a translation product of a gene coding for the activator protein for vesicle secretion shown in SEQ ID NO: 1, and/or
(iii) a fragment, or derivative, or variant of said transcription or translation product,

in a sample obtained from a subject not suffering from said neurodegenerative disease.

**7.** The method according to any of claims 1 to 6 wherein an alteration in mRNA and/or protein of the gene coding for the activator protein for vesicle secretion shown in SEQ ID NO: 1, in a cell, or tissue, or body fluid obtained from said subject relative to a reference value representing a known health status indicates a diagnosis, or prognosis, or increased risk of Alzheimer's disease in said subject.

**8.** A kit for diagnosing or prognosticating a neurodegenerative disease, in particular Alzheimer's disease, in a subject, or determining the propensity or predisposition of a subject to develop such a disease by:

(i) detecting in a sample obtained from said subject a level, or an activity, or both said level and said activity of a transcription product and/or of a translation product of a gene coding for the activator protein for vesicle secretion shown in SEQ ID NO: 1 compared to a reference value representing a known health status; and said kit comprising:

a) at least one reagent which is selected from the group consisting of (i) reagents that selectively detect a transcription product of a gene coding for the activator protein for vesicle secretion shown in SEQ ID NO: 1 and (ii) reagents that selectively detect a translation product of a gene coding for the activator protein for vesicle secretion shown in SEQ ID NO: 1.

**9.** A recombinant, non-human animal comprising a non-native gene sequence coding for the activator protein for vesicle secretion shown in SEQ ID NO: 1, or a fragment, or a derivative, or a variant thereof, said animal being obtainable by:

(i) providing a gene targeting construct comprising said gene sequence and a selectable marker sequence, and
(ii) introducing said targeting construct into a stem cell of a non-human animal, and
(iii) introducing said non-human animal stem cell into a non-human embryo, and
(iv) transplanting said embryo into a pseudopregnant non-human animal, and
(v) allowing said embryo to develop to term, and
(vi) identifying a genetically altered non-human animal whose genome comprises a modification of said gene sequence in both alleles, and
(vii) breeding the genetically altered non-human animal of step (vi) to obtain a genetically altered non-human animal whose genome comprises a modification of said endogenous gene, wherein said disruption results in said non-human animal exhibiting a predisposition to developing a neurodegenerative disease or related diseases or disorders.

**10.** Use of the recombinant, non-human animal according to claim 9 for screening, testing, and validating compounds, agents, and modulators in the development of diagnostics and therapeutics to treat neurodegenerative diseases, in particular Alzheimer's disease.

**11.** An assay for screening for a modulator of neurodegenerative diseases, in particular Alzhelmer's disease, or related diseases or disorders of one or more substances selected from the group consisting of

(i) a gene coding for the activator protein for vesicle secretion shown in SEQ ID NO: 1, and/or
(ii) a transcription product of a gene coding for the activator protein for vesicle secretion shown in SEQ ID NO: 1, and/or
(iii) a translation product of a gene coding for the activator protein for vesicle secretion shown in SEQ ID NO: 1, and/or
(iv) a fragment, or derivative, or variant of (i) to (iii), said method comprising:

a) contacting a cell with a test compound;
b) measuring the activity and/or level of one or more substances recited in (i) to (iv);
c) measuring the activity and/or level of one or more substances recited in (i) to (iv) in a control cell not contacted with said test compound; and
d) comparing the levels and/or activities of the substance in the cells of step (b) and (c), wherein an alteration in the activity and/or level of substances in the contacted cells indicates that the test compound is a modulator of said diseases or disorders.

**12.** In vitro use of a protein molecule of SEQ ID NO: 1, said protein molecule being a translation product of the gene coding for the activator protein for vesicle secretion, or a fragment, or derivative, or variant thereof, as a diagnostic target for detecting a neurodegenerative disease, preferably Alzheimer's disease.

**13.** Use of a protein molecule of SEQ ID NO: 1, said protein molecule being a translation product of the gene coding for the activator protein for vesicle secretion, or a fragment, or derivative, or variant thereof, as a screening target for reagents or compounds preventing, or treating, or ameliorating a neurodegenerative disease, preferably Alzheimer's disease.

**14.** Use of an antibody specifically immunoreactive with an immunogen, wherein said immunogen is a translation product of the gene coding for the activator protein for vesicle secretion shown in SEQ ID NO: 1, or a fragment, or derivative, or variant thereof, for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell.

**Revendications**

**1.** Procédé pour diagnostiquer ou pronostiquer une maladie neurodégénérative chez un sujet, ou pour déterminer si un sujet présente un risque accru de développer ladite maladie, comprenant :

la détermination d'un taux et/ou d'une activité de

(i) un produit de transcription d'un gène codant pour la protéine activatrice de sécrétion vésiculaire représentée dans SEQ ID N° 1, et/ou
(ii) un produit de traduction d'un gène codant pour la protéine activatrice de sécrétion vésiculaire représentée dans SEQ ID N° 1, et/ou
(iii) un fragment ou dérivé ou variant dudit produit de transcription ou de traduction,

dans un échantillon provenant dudit sujet et la comparaison dudit taux et/ou de ladite activité à une valeur de référence représentant un état pathologique ou sain connu, pour ainsi diagnostiquer ou pronostiquer ladite maladie neurodégénérative chez ledit sujet, ou déterminer si ledit sujet présente un risque accru de développer ladite maladie neurodégénérative.

**2.** Procédé pour contrôler l'évolution d'une maladie neurodégénérative chez un sujet, comprenant :

la détermination d'un taux et/ou d'une activité de

(i) un produit de transcription d'un gène codant pour la protéine activatrice de sécrétion vésiculaire représentée dans SEQ ID N° 1, et/ou
(ii) un produit de traduction d'un gène codant pour la protéine activatrice de sécrétion vésiculaire représentée dans la SEQ ID N° 1, et/ou

(iii) un fragment ou dérivé ou variant dudit produit de transcription ou de traduction,

dans un échantillon provenant dudit sujet et la comparaison dudit taux et/ou de ladite activité à une valeur de référence représentant un état pathologique ou sain connu, pour contrôler ainsi l'évolution de ladite maladie neurodégénérative chez ledit sujet.

**3.** Procédé d'évaluation d'un traitement d'une maladie neurodégénérative, comprenant :

la détermination d'un taux et/ou d'une activité de

(i) un produit de transcription d'un gène codant pour la protéine activatrice de sécrétion vésiculaire représentée dans SEQ ID N° 1, et/ou
(ii) un produit de traduction d'un gène codant pour la protéine activatrice de sécrétion vésiculaire représentée dans SEQ ID N° 1, et/ou
(iii) un fragment ou dérivé ou variant dudit produit de transcription ou de traduction,

dans un échantillon provenant d'un sujet soumis à un traitement pour ladite maladie et la comparaison dudit taux et/ou de ladite activité à une valeur de référence représentant un état pathologique ou sain connu, pour évaluer ainsi ledit traitement de ladite maladie neurodégénérative.

**4.** Procédé selon l'une quelconque des revendications 1 à 3, dans lequel ladite maladie neurodégénérative est la maladie d'Alzheimer.

**5.** Procédé selon l'une quelconque des revendications 1 à 4, dans lequel ledit échantillon comporte une cellule ou un tissu ou un organe ou un fluide corporel, en particulier du liquide céphalo-rachidien ou du sang.

**6.** Procédé selon l'une quelconque des revendications 1 à 5, dans lequel ladite valeur de référence est celle d'un taux et/ou d'une activité de

(i) un produit de transcription d'un gène codant pour la protéine activatrice de sécrétion vésiculaire représentée dans SEQ ID N° 1, et/ou
(ii) un produit de traduction d'un gène codant pour la protéine activatrice de sécrétion vésiculaire représentée dans la SEQ ID N° 1, et/ou
(iii) un fragment ou dérivé ou variant dudit produit de transcription ou de traduction,

dans un échantillon provenant d'un sujet ne souffrant pas de ladite maladie neurodégénérative.

**7.** Procédé selon l'une quelconque des revendications 1 à 6, dans lequel une altération dans l'ARNm et/ou la protéine du gène codant pour la protéine activatrice de sécrétion vésiculaire représentée dans SEQ ID N° 1, dans une cellule ou un tissu ou un fluide corporel provenant dudit sujet, par rapport à une valeur de référence représentant un état de santé connu, indique un diagnostic ou un pronostic ou un risque accru de maladie d'Alzheimer chez ledit sujet.

**8.** Trousse pour diagnostiquer ou pronostiquer une maladie neurodégénérative, en particulier la maladie d'Alzheimer, chez un sujet, ou déterminer la tendance ou la prédisposition d'un sujet à développer une telle maladie par :

(i) détection dans un échantillon provenant dudit sujet d'un taux ou d'une activité, ou à la fois dudit taux et de ladite activité, d'un produit de transcription et/ou d'un produit de traduction d'un gène codant pour la protéine activatrice de sécrétion vésiculaire représentée dans SEQ ID N° 1 comparativement à une valeur de référence représentant un état de santé connu ; et ladite trousse comprenant :

a) au moins un réactif qui est choisi dans le groupe formé par (i) les réactifs qui détectent sélectivement un produit de transcription d'un gène codant pour la protéine activatrice de sécrétion vésiculaire représentée dans SEQ ID N° 1 et (ii) les réactifs qui détectent sélectivement un produit de traduction d'un gène codant pour la protéine activatrice de sécrétion vésiculaire représentée dans SEQ ID N° 1.

**9.** Animal non humain recombinant comprenant une séquence génique non native codant pour la protéine activatrice de sécrétion vésiculaire représentée dans SEQ ID N° 1, ou un fragment ou un dérivé ou un variant de celle-ci, ledit animal pouvant être obtenu par les étapes consistant à :

(i) fournir une construction ciblante génique comprenant ladite séquence génique et une séquence de marqueur sélectionnable, et
(ii) introduire ladite construction ciblante dans une cellule souche d'un animal non humain, et
(iii) introduire ladite cellule souche d'animal non humain dans un embryon non humain, et
(iv) transplanter ledit embryon dans un animal non humain pseudogravide, et
(v) laisser ledit embryon se développer à terme, et
(vi) identifier un animal non humain génétiquement altéré dont le génome comprend une modification de ladite séquence génique dans les deux allèles, et
(vii) élever l'animal non humain génétiquement altéré de l'étape (vi) pour obtenir un animal non humain génétiquement altéré dont le génome comprend une modification dudit gène endogène, cette disruption ayant pour résultat que ledit animal non humain présente une prédisposition à développer une maladie neurodégénérative ou des maladies ou troubles associés.

**10.** Utilisation d'un animal non humain recombinant selon la revendication 9, pour le criblage, le test et la validation de composés, agents et modulateurs dans le développement de diagnostics et thérapies pour traiter des maladies neurodégénératives, en particulier la maladie d'Alzheimer.

**11.** Essai de criblage pour sélectionner un modulateur de maladies neurodégénératives, en particulier de la maladie d'Alzheimer, ou de maladies ou troubles associés agissant sur une ou plusieurs substances choisies dans le groupe formé par

(i) un gène codant pour la protéine activatrice de sécrétion vésiculaire représentée dans SEQ ID N° 1, et/ou
(ii) un produit de transcription d'un gène codant pour la protéine activatrice de sécrétion vésiculaire représentée dans SEQ ID N° 1, et/ou
(iii) un produit de traduction d'un gène codant pour la protéine activatrice de sécrétion vésiculaire représentée dans SEQ ID N° 1, et/ou
(iv) un fragment ou un dérivé ou un variant de (i) à (iii),

ledit procédé comprenant les étapes consistant à :

a) mettre en contact une cellule avec un composé à tester ;
b) mesurer l'activité et/ou le taux d'une ou plusieurs substances citées en (i) à (iv) ;
c) mesurer l'activité et/ou le taux d'une ou plusieurs substances citées en (i) à (iv) dans une cellule témoin n'ayant pas été mise en contact avec ledit composé à tester ; et
d) comparer les taux et/ou les activités de la substance dans les cellules de l'étape (b) et de l'étape (c), une altération de l'activité et/ou du taux des substances dans les cellules mises en contact indiquant que le composé à tester est un modulateur desdites maladies ou des desdits troubles.

**12.** Utilisation *in vitro* d'une molécule protéique de SEQ ID N° 1, ladite molécule protéique étant un produit de traduction du gène codant pour la protéine activatrice de sécrétion vésiculaire, ou un fragment ou un dérivé ou un variant de celle-ci, comme cible diagnostique pour détecter une maladie neurodégénérative, de préférence la maladie d'Alzheimer.

**13.** Utilisation d'une molécule protéique de SEQ ID N° 1, ladite molécule protéique étant un produit de traduction du gène codant pour la protéine activatrice de sécrétion vésiculaire, ou un fragment ou un dérivé ou un variant de celle-ci, comme cible de criblage de réactifs ou composés prévenant ou traitant ou améliorant une maladie neurodégénérative, de préférence la maladie d'Alzheimer.

**14.** Utilisation d'un anticorps spécifiquement immunoréactif avec un immunogène, dans laquelle ledit immunogène est un produit de traduction du gène codant pour la protéine activatrice de sécrétion vésiculaire représentée dans SEQ ID N° 1, ou un fragment ou un dérivé ou un variant de celle-ci, pour détecter l'état pathologique d'une cellule dans un échantillon provenant d'un sujet, comprenant la coloration immunocytochimique de ladite cellule avec ledit anticorps, où un degré altéré de coloration ou un aspect altéré de coloration dans ladite cellule comparée à une cellule représentant un état de santé connu indique un état pathologique de ladite cellule.

## Patentansprüche

1. Verfahren zum Diagnostizieren oder Prognostizieren einer neurodegenerativen Krankheit bei einem Patienten oder zum Bestimmen, ob ein Patient ein erhöhtes Risiko hat, diese Krankheit zu entwickeln, umfassend:

   Bestimmen einer Konzentration und/oder einer Aktivität von:

   (i) einem Transcriptionsprodukt eines Gens, das für das Aktivatorprotein für die Vesikelsekretion codiert, wie es in SEQ ID Nr. 1 gezeigt ist; und/oder
   (ii) einem Translationsprodukt eines Gens, das für das Aktivatorprotein für die Vesikelsekretion codiert, wie es in SEQ ID Nr. 1 gezeigt ist; und/oder
   (iii) einem Fragment oder Derivat oder einer Variante des Transcriptions- oder Translationsprodukts;

   in einer Probe, die von dem Patienten erhalten wurde, und Vergleichen der Konzentration und/oder der Aktivität mit einem Referenzwert, der einen bekannten Krankheits- oder Gesundheitszustand darstellt, wodurch die neurodegenerative Krankheit bei dem Patienten diagnostiziert oder prognostiziert wird oder bestimmt wird, ob der Patient ein erhöhtes Risiko hat, diese neurodegenerative Krankheit zu entwickeln.

2. Verfahren zur Überwachung des Fortschritts einer neurodegenerativen Krankheit bei einem Patienten, umfassend:

   Bestimmen einer Konzentration und/oder einer Aktivität von:

   (i) einem Transcriptionsprodukt eines Gens, das für das Aktivatorprotein für die Vesikelsekretion codiert, wie es in SEQ ID Nr. 1 gezeigt ist; und/oder
   (ii) einem Translationsprodukt eines Gens, das für das Aktivatorprotein für die Vesikelsekretion codiert, wie es in SEQ ID Nr. 1 gezeigt ist; und/oder
   (iii) einem Fragment oder Derivat oder einer Variante des Transcriptions- oder Translationsprodukts;

   in einer Probe, die von dem Patienten erhalten wurde, und Vergleichen der Konzentration und/oder der Aktivität mit einem Referenzwert, der einen bekannten Krankheits- oder Gesundheitszustand darstellt, wodurch der Fortschritt der neurodegenerativen Krankheit bei dem Patienten überwacht wird.

3. Verfahren zur Bewertung einer Behandlung auf eine neurodegenerative Krankheit, umfassend:

   Bestimmen einer Konzentration und/oder einer Aktivität von:

   (i) einem Transcriptionsprodukt eines Gens, das für das Aktivatorprotein für die Vesikelsekretion codiert, wie es in SEQ ID Nr. 1 gezeigt ist; und/oder
   (ii) einem Translationsprodukt eines Gens, das für das Aktivatorprotein für die Vesikelsekretion codiert, wie es in SEQ ID Nr. 1 gezeigt ist; und/oder
   (iii) einem Fragment oder Derivat oder einer Variante des Transcriptions- oder Translationsprodukts;

   in einer Probe, die von einem Patienten erhalten wurde, der auf die Krankheit behandelt wird, und Vergleichen der Konzentration und/oder der Aktivität mit einem Referenzwert, der einen bekannten Krankheits- oder Gesundheitszustand darstellt, wodurch die Behandlung auf die neurodegenerative Krankheit bewertet wird.

4. Verfahren gemäß einem der Ansprüche 1 bis 3, wobei es sich bei der neurodegenerativen Krankheit um die Alzheimer-Krankheit handelt.

5. Verfahren gemäß einem der Ansprüche 1 bis 4, wobei die Probe eine Zelle oder ein Gewebe oder ein Organ oder eine Körperflüssigkeit, insbesondere Liquor oder Blut, umfasst.

6. Verfahren gemäß einem der Ansprüche 1 bis 5, wobei der Referenzwert der Referenzwert einer Konzentration und/oder einer Aktivität von

   (i) einem Transcriptionsprodukt eines Gens, das für das Aktivatorprotein für die Vesikelsekretion codiert, wie es in SEQ ID Nr. 1 gezeigt ist; und/oder
   (ii) einem Translationsprodukt eines Gens, das für das Aktivatorprotein für die Vesikelsekretion codiert, wie es in SEQ ID Nr. 1 gezeigt ist; und/oder
(iii) einem Fragment oder Derivat oder einer Variante des Transcriptions- oder Translationsprodukts;

in einer Probe von einem Patienten, der nicht an der neurodegenerativen Krankheit leidet, ist.

**7.** Verfahren gemäß einem der Ansprüche 1 bis 6, wobei eine Veränderung in der mRNA und/oder im Protein des Gens, das für das Aktivatorprotein für die Vesikelsekretion codiert, wie es in SEQ ID Nr. 1 gezeigt ist, in einer Zelle oder einem Gewebe oder einer Körperflüssigkeit, die bzw. das von dem Patienten erhalten wurde, relativ zu einem Referenzwert, der einen bekannten Gesundheitszustand darstellt, eine Diagnose oder Prognose oder ein erhöhtes Risiko von Alzheimer-Krankheit bei dem Patienten anzeigt.

**8.** Kit zum Diagnostizieren oder Prognostizieren einer neurodegenerativen Krankheit, insbesondere Alzheimer-Krankheit, bei einem Patienten oder zum Bestimmen der Neigung oder Prädisposition eines Patienten, eine solche Krankheit zu entwickeln, durch:

(i) Nachweisen einer Konzentration oder einer Aktivität oder sowohl einer Konzentration als auch einer Aktivität eines Transcriptionsprodukts und/oder eines Translationsprodukts eines Gens, das für das Aktivatorprotein für die Vesikelsekretion codiert, wie es in SEQ ID Nr. 1 gezeigt ist, in einer Probe, die von dem Patienten erhalten wurde, im Vergleich zu einem Referenzwert, der einen bekannten Gesundheitszustand darstellt; wobei der Kit Folgendes umfasst:

a) wenigstens ein Reagens, das aus der Gruppe ausgewählt ist, die aus Folgendem besteht: (i) Reagentien, die selektiv ein Transcriptionsprodukt eines Gens, das für das Aktivatorprotein für die Vesikelsekretion codiert, wie es in SEQ ID Nr. 1 gezeigt ist, nachweisen, und (ii) Reagentien, die selektiv ein Translationsprodukt eines Gens, das für das Aktivatorprotein für die Vesikelsekretion codiert, wie es in SEQ ID Nr. 1 gezeigt ist, nachweisen.

**9.** Rekombinantes nichthumanes Tier, das eine nichtnative Gensequenz, die für das Aktivatorprotein für die Vesikelsekretion codiert, wie es in SEQ ID Nr. 1 gezeigt ist, oder ein Fragment oder Derivat oder eine Variante davon umfasst, wobei das Tier erhältlich ist durch:

(i) Bereitstellen eines Genzielsteuerungskonstrukts, das die Gensequenz und eine Selektionsmarkersequenz umfasst; und
(ii) Einführen des Zielsteuerungskonstrukts in eine Stammzelle eines nichthumanen Tiers; und
(iii) Einführen der Stammzelle des nichthumanen Tiers in einen nichthumanen Embryo; und
(iv) Transplantieren des Embryos in ein pseudoträchtiges nichthumanes Tier; und
(v) Entwickelnlassen des Embryos bis zur Reife; und
(vi) Identifizieren eines genetisch veränderten nichthumanen Tiers, dessen Genom eine Modifikation der Gensequenz in beiden Allelen umfasst; und
(vii) Aufziehen des genetisch veränderten nichthumanen Tiers von Schritt (vi), so dass man ein genetisch verändertes nichthumanes Tier erhält, dessen Genom eine Modifikation des endogenen Gens umfasst, wobei der Eingriff dazu führt, dass das nichthumane Tier eine Prädisposition aufweist, eine neurodegenerative Krankheit oder verwandte Krankheiten oder Störungen zu entwickeln.

**10.** Verwendung des rekombinanten nichthumanen Tiers gemäß Anspruch 9 zum Durchmustern, Testen und Bewerten von Verbindungen, Mitteln und Modulatoren bei der Entwicklung von Diagnostika und Therapeutika zur Behandlung von neurodegenerativen Krankheiten, insbesondere Alzheimer-Krankheit.

**11.** Assay zum Suchen nach einem Modulator von neurodegenerativen Krankheiten, insbesondere Alzheimer-Krankheit, oder verwandten Krankheiten oder Störungen durch Screening von einer oder mehreren Substanzen, die aus der Gruppe ausgewählt sind, die aus Folgenden besteht:

(i) einem Gen, das für das Aktivatorprotein für die Vesikelsekretion codiert, wie es in SEQ ID Nr. 1 gezeigt ist; und/oder
(ii) einem Transcriptionsprodukt eines Gens, das für das Aktivatorprotein für die Vesikelsekretion codiert, wie es in SEQ ID Nr. 1 gezeigt ist; und/oder
(iii) einem Translationsprodukt eines Gens, das für das Aktivatorprotein für die Vesikelsekretion codiert, wie es in SEQ ID Nr. 1 gezeigt ist; und/oder

(iv) einem Fragment oder Derivat oder einer Variante von (i) bis (iii);

wobei das Verfahren Folgendes umfasst:

a) In-Kontakt-Bringen einer Zelle mit einer Testverbindung;
b) Messen der Aktivität und/oder der Konzentration von einer oder mehreren Substanzen, die in (i) bis (iv) genannt sind;
c) Messen der Aktivität und/oder der Konzentration von einer oder mehreren Substanzen, die in (i) bis (iv) genannt sind, in einer Kontrollzelle, die nicht mit der Testverbindung in Kontakt gebracht wurde; und
d) Vergleichen der Konzentrationen und/oder Aktivitäten der Substanz in den Zellen der Schritte (b) und (c), wobei eine Änderung der Aktivität und/oder der Konzentration der Substanzen in den kontaktierten Zellen anzeigt, dass die Testverbindung ein Modulator der Krankheiten oder Störungen ist.

**12.** In-vitro-Verwendung eines Proteinmoleküls von SEQ ID Nr. 1, wobei das Proteinmolekül ein Translationsprodukt des Gens ist, das für das Aktivatorprotein für die Vesikelsekretion codiert, oder eines Fragments oder Derivats oder einer Variante davon als diagnostisches Target zum Nachweis einer neurodegenerativen Krankheit, vorzugsweise Alzheimer-Krankheit.

**13.** Verwendung eines Proteinmoleküls von SEQ ID Nr. 1, wobei das Proteinmolekül ein Translationsprodukt des Gens ist, das für das Aktivatorprotein für die Vesikelsekretion codiert, oder eines Fragments oder Derivats oder einer Variante davon als Screening-Target für Reagentien oder Verbindungen, die eine neurodegenerative Krankheit, vorzugsweise Alzheimer-Krankheit, verhindern oder behandeln oder lindern.

**14.** Verwendung eines Antikörpers, der gegenüber einem Immunogen spezifisch immunreaktiv ist, wobei das Immunogen ein Translationsprodukt des Gens, das für das Aktivatorprotein für die Vesikelsekretion codiert, wie es in SEQ ID Nr. 1 gezeigt ist, oder ein Fragments oder Derivat oder eine Variante davon ist, zum Nachweis des pathologischen Zustands einer Zelle in einer Probe, die von einem Patienten erhalten wurde, der das immunocytochemische Anfärben der Zelle mit dem Antikörper umfasst, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle anzeigt.

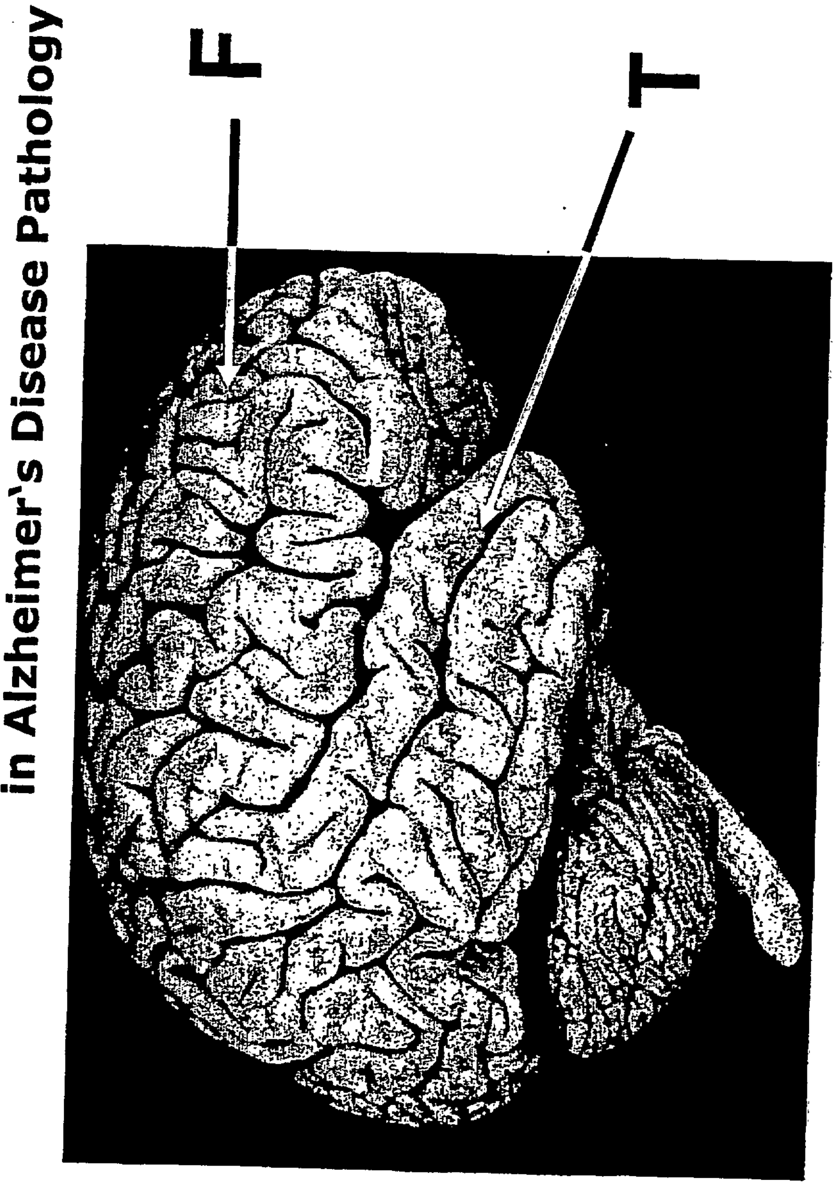


Fig. 1: Identification of Genes Involved in Alzheimer's Disease Pathology

Fig. 2: Identification of differentially expressed genes in a suppressive subtractive hybridization screen by dot blot analysis

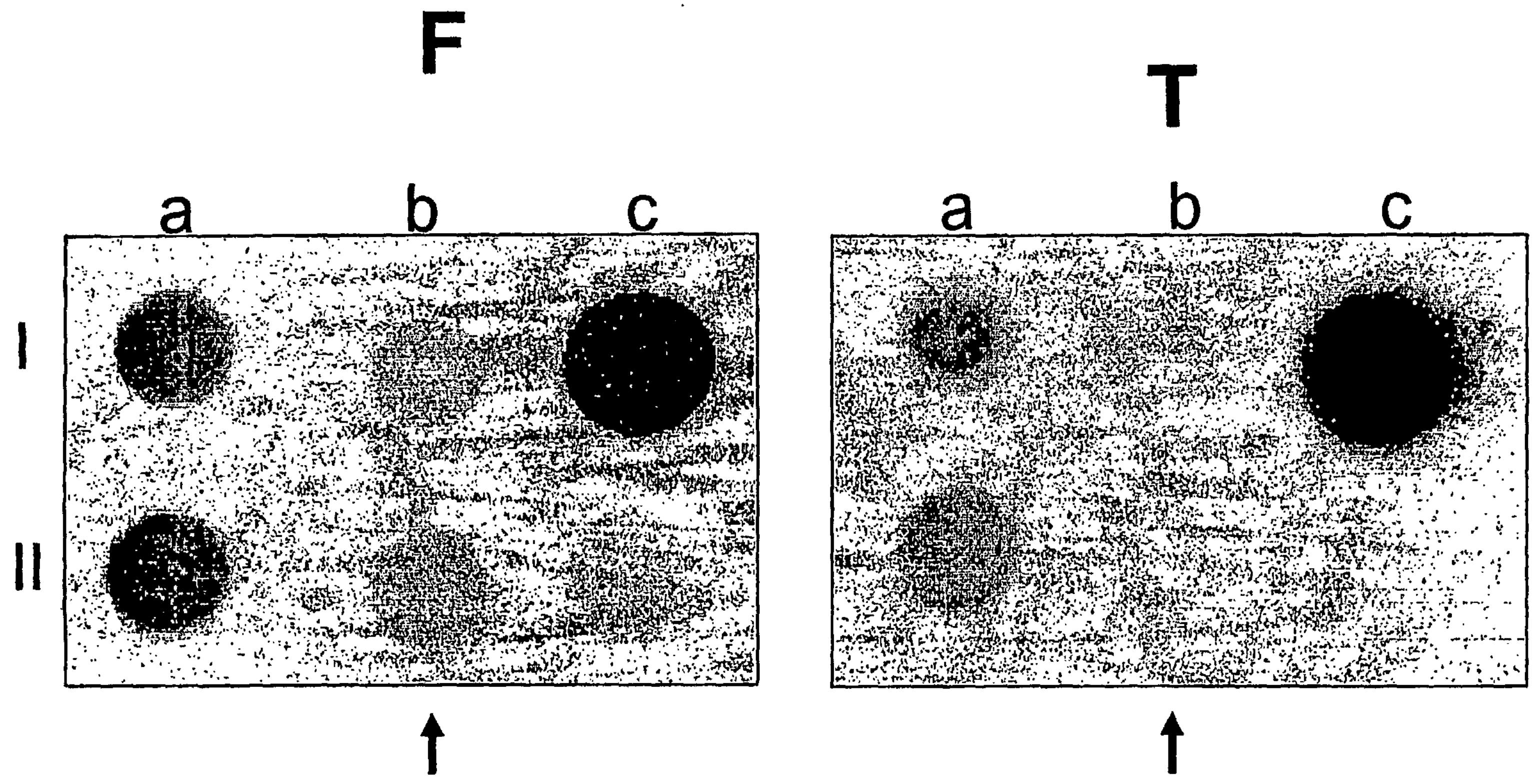

**Fig. 3: Verification of differential expression of CAPS by quantitative PCR**

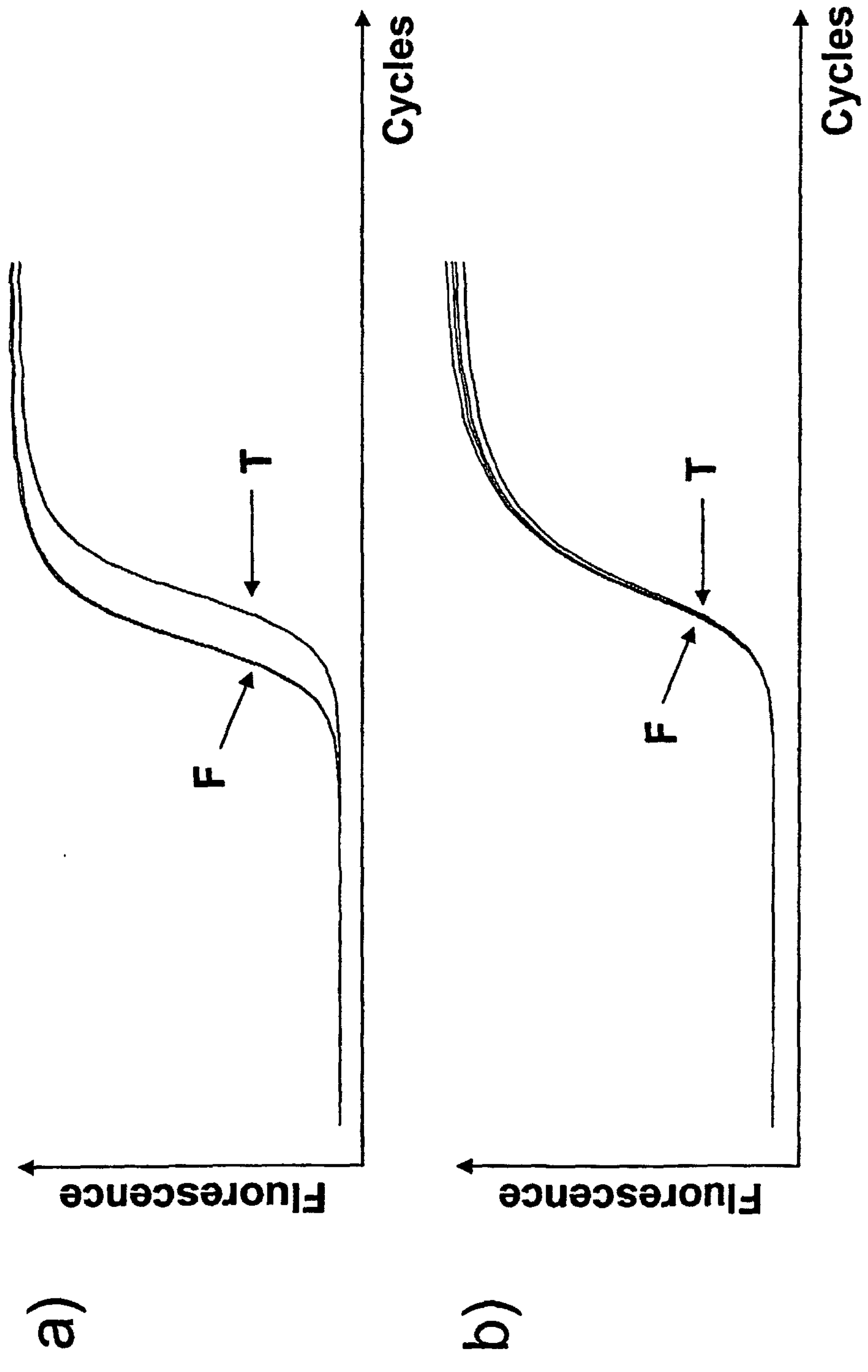

# Fig. 4: SEQ ID NO. 1: amino acid sequence of human CAPS protein

**Length: 1353 aa**

```
   1 MLDPSSSEEE SDEIVEEESG KEVLGSAPSG ARLSPSRTSE GSAGSAGLGG
  51 GGAGAGAGVG AGGGGGSGAS SGGGAGGLQP SSRAGGGRPS SPSPSVVSEK
 101 EKEELERLQK EEEERKKRLQ LYVFVMRCIA YPFNAKQPTD MARRQQKISK
 151 QQLQTVKDRF QAFLNGETQI MADEAFMNAV QSYYEVFLKS DRVARMVQSG
 201 GCSANDSREV FKKHIEKRVR SLPEIDGLSK ETVLSSWMAK FDAIYRGEED
 251 PRKQQARMTA SAASELILSK EQLYEMFQNI LGIKKFEHQL LYNACQLDNP
 301 DEQAAQIRRE LDGRLQMADQ IARERKFPKF VSKEMENMYI EELKSSVNLL
 351 MANLESMPVS KGGEFKLQKL KRSHNASIID MGEESENQLS KSDVVLSFSL
 401 EVVIMEVQGL KSLAPNRIVY CTMEVEGGEK LQTDQAEASK PTWGTQGDFS
 451 TTHALPAVKV KLFTESTGVL ALEDKELGRV ILHPTPNSPK QSEWHKMTVS
 501 KNCPDQDLKI KLAVRMDKPQ NMKHSGYLWA IGKNVWKRWK KRFFVLVQVS
 551 QYTFAMCSYR EKKAEPQELL QLDGYTVDYT DPQPGLEGGR AFFNAVKEGD
 601 TVIFASDDEQ DRILWVQAMY RATGQSHKPV PPTQVQKLNA KGGNVPQLDA
 651 PISQFYADRA QKHGMDEFIS SNPCNFDHAS LFEMVQRLTL DHRLNDSYSC
 701 LGWFSPGQVF VLDEYCARNG VRGCHRHLCY LRDLLERAEN GAMIDPTLLH
 751 YSFAFCASHV HGNRPDGIGT VTVEEKERFE EIKERLRVLL ENQITHFRYC
 801 FPFGRPEGAL KATLSLLERV LMKDIVTPVP QEEVKTVIRK CLEQAALVNY
 851 SRLSEYAKIE ENQKDAENVG RLITPAKKLE DTIRLAELVI EVLQQNEEHH
 901 AEPHVDKGEA FAWWSDLMVE HAETFLSLFA VDMDAALEVQ PPDTWDSFPL
 951 FQLLNDFLRT DYNLCNGKFH KHLQDLFAPL VVRYVDLMES SIAQSIHRGF
1001 ERESWEPVKS LTSNLPNVNL PNVNLPKVPN LPVNIPLGIP QMPTFSAPSW
1051 MAAIYDADNG SGTSEDLFWK LDALQTFIRD LHWPEEEFGK HLEQRLKLMA
1101 SDMIESCVKR TRIAFEVKLQ KTSRSTDFRV PQSICTMFNV MVDAKAQSTK
1151 LCSMEMGQEH QYHSKIDELI EETVKEMITL LVAKFVTILE GVLAKLSRYD
1201 EGTLFSSFLS FTVKAASKYV DVPKPGMDVA DAYVTFVRHS QDVLRDKVNE
1251 EMYIERLFDQ WYNSSMNVIC TWLTDRMDLQ LHIYQLKTLI RVVKKTYRDF
1301 RLQGVLDSTL NSKTYETIRN RLTVEEATAS VSEGGGLQGI SMKDSDEEDE
1351 EDD
```

# Fig. 5: Nucleotide sequence of SEQ ID NO: 2

**Length: 641 bp**

```
  1  CATATTTGGA AGCTGCCTTC ACGGTAAATG ACAGAAAAGA AGAAAACAAA
 51  GTCCCTTCGT CATATCTGGA TAATTTTGCC AGCACTCCTT CCAAGATAGT
101  AACGAACTTT GCAACCAAGA GTGTTATCAT TTCTTTAACA GTTTCTTCAA
151  TTAGTTCGTC TATTTTTGAA TGGTATTGAT GCTCTTGGCC CATTTCCATG
201  CTGCAAAGTT TTGTTGATTG AGCTTTGGCA TCAACCATAA CATTAAACAT
251  GGTGCATATT GACTGTGGGA CTCGAAAATC TGTTGATCGA CTGGTTTTTT
301  GCAGCTTAAC TTCAAATGCA ATCCTGGTTC TTTTGACACA AGATTCGATC
351  ATGTCACTTG CCATCAGCTT CAGCCGTTGT TCCAGGTGCT TTCCAAACTC
401  TTCTTCAGGC CAGTGCAGGT CCCGAATGAA GGTCTGAAGG GCGTCAAGTT
451  TCCAAAACAG ATCTTCTGAG GTGCCTGACC CATTATTGAC TGGTTCCCAT
501  GACTCCCGCT CAAAGCCCCT GTGAATGGAT TGTGCAATTG AGGACTCCAT
551  CAGATCCACA TATCTAACAA CAAGTGGGGC AAACAGGTCT TGCAGGTGTT
601  TGTGAAATTT TCCATTGCAC AAATTATAGT CAGTACCTCG G
```

# Fig. 6: Alignment of SEQ ID NO: 2 with human CAPS cDNA KIAA 1121 (AB032947)

**Length: 783 bp; Gaps: 1; Percent Identity: 99.843**

```
 636 GTACTGACTATAATTTGTGCAATGGAAAATTTCACAAACACCTGCAAGAC 587
     ||||||||||||||||||||||||||||||||||||||||||||||||||
3226 GTACTGACTATAATTTGTGCAATGGAAAATTTCACAAACACCTGCAAGAC 3275

 586 CTGTTTGCCCCACTTGTTGTTAGATATGTGGATCTGATGGAGTCCTCAAT 537
     ||||||||||||||||||||||||||||||||||||||||||||||||||
3276 CTGTTTGCCCCACTTGTTGTTAGATATGTGGATCTGATGGAGTCCTCAAT 3325

 536 TGCACAATCCATTCACAGGGGCTTTGAGCGGGAGTCATGGGAACCAGTCA 487
     ||||||||||||||||||||||||||||||||||||||||||||||||||
3326 TGCACAATCCATTCACAGGGGCTTTGAGCGGGAGTCATGGGAACCAGTCA 3375
                               .
                               .
                               .

 486 ...............................................ATA 484
                                                    |||
3476 GCCTACTTTTTCGGCACCGTCATGGATGGCTGCTATATATGATGCGGATA 3525

 483 ATGGGTCAGGCACCTCAGAAGATCTGTTTTGGAAACTTGACGCCCTTCAG 434
     ||||||||||||||||||||||||||||||||||||||||||||||||||
3526 ATGGGTCAGGCACCTCAGAAGATCTGTTTTGGAAACTTGACGCCCTTCAG 3575

 433 ACCTTCATTCGGGACCTGCACTGGCCTGAAGAAGAGTTTGGAAAGCACCT 384
     ||||||||||||||||||||||||||||||||||||||||||||||||||
3576 ACCTTCATTCGGGACCTGCACTGGCCTGAAGAAGAGTTTGGAAAGCACCT 3625

 383 GGAACAACGGCTGAAGCTGATGGCAAGTGACATGATCGAATCTTGTGTCA 334
     |||||||||||||||| |||||||||||||||||||||||||||||||||
3626 GGAACAACGGCTGAAGTTGATGGCAAGTGACATGATCGAATCTTGTGTCA 3675

 333 AAAGAACCAGGATTGCATTTGAAGTTAAGCTGCAAAAAACCAGTCGATCA 284
     ||||||||||||||||||||||||||||||||||||||||||||||||||
3676 AAAGAACCAGGATTGCATTTGAAGTTAAGCTGCAAAAAACCAGTCGATCA 3725

 283 ACAGATTTTCGAGTCCCACAGTCAATATGCACCATGTTTAATGTTATGGT 234
     ||||||||||||||||||||||||||||||||||||||||||||||||||
3726 ACAGATTTTCGAGTCCCACAGTCAATATGCACCATGTTTAATGTTATGGT 3775

 233 TGATGCCAAAGCTCAATCAACAAAACTTTGCAGCATGGAAATGGGCCAAG 184
     ||||||||||||||||||||||||||||||||||||||||||||||||||
3776 TGATGCCAAAGCTCAATCAACAAAACTTTGCAGCATGGAAATGGGCCAAG 3825

 183 AGCATCAATACCATTCAAAAATAGACGAACTAATTGAAGAAACTGTTAAA 134
     ||||||||||||||||||||||||||||||||||||||||||||||||||
3826 AGCATCAATACCATTCAAAAATAGACGAACTAATTGAAGAAACTGTTAAA 3875

 133 GAAATGATAACACTCTTGGTTGCAAAGTTCGTTACTATCTTGGAAGGAGT 84
     ||||||||||||||||||||||||||||||||||||||||||||||||||
3876 GAAATGATAACACTCTTGGTTGCAAAGTTCGTTACTATCTTGGAAGGAGT 3925

  83 GCTGGCAAAATTATCCAGATATGACGAAGGGACTTTGTTTTCTTCTTTTC 34
     ||||||||||||||||||||||||||||||||||||||||||||||||||
3926 GCTGGCAAAATTATCCAGATATGACGAAGGGACTTTGTTTTCTTCTTTTC 3975

  33 TGTCATTTACCGTGAAGGCAGCTTCCAAATATG 1
     |||||||||||||||||||||||||||||||||
3976 TGTCATTTACCGTGAAGGCAGCTTCCAAATATG 4008
```

**Fig. 7: Schematic alignment of SEQ ID NO: 2 with human CAPS cDNA KIAA1121 (accession number AB032947)**

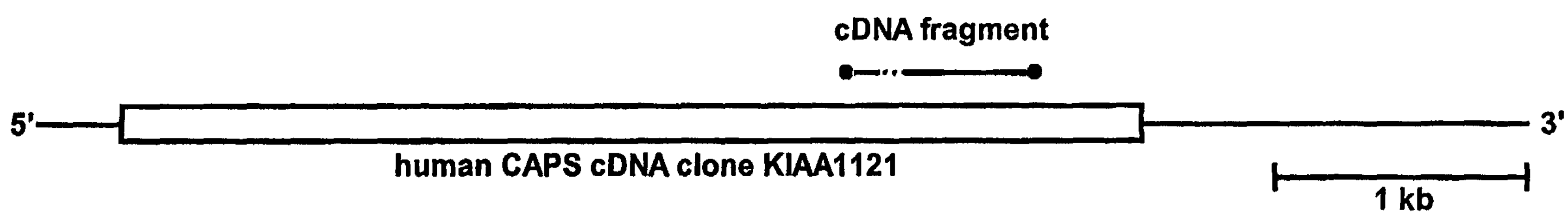

**Fig. 8: Images of the human cerebral cortex labeled with anti-CAPS antibody and with DAPI**

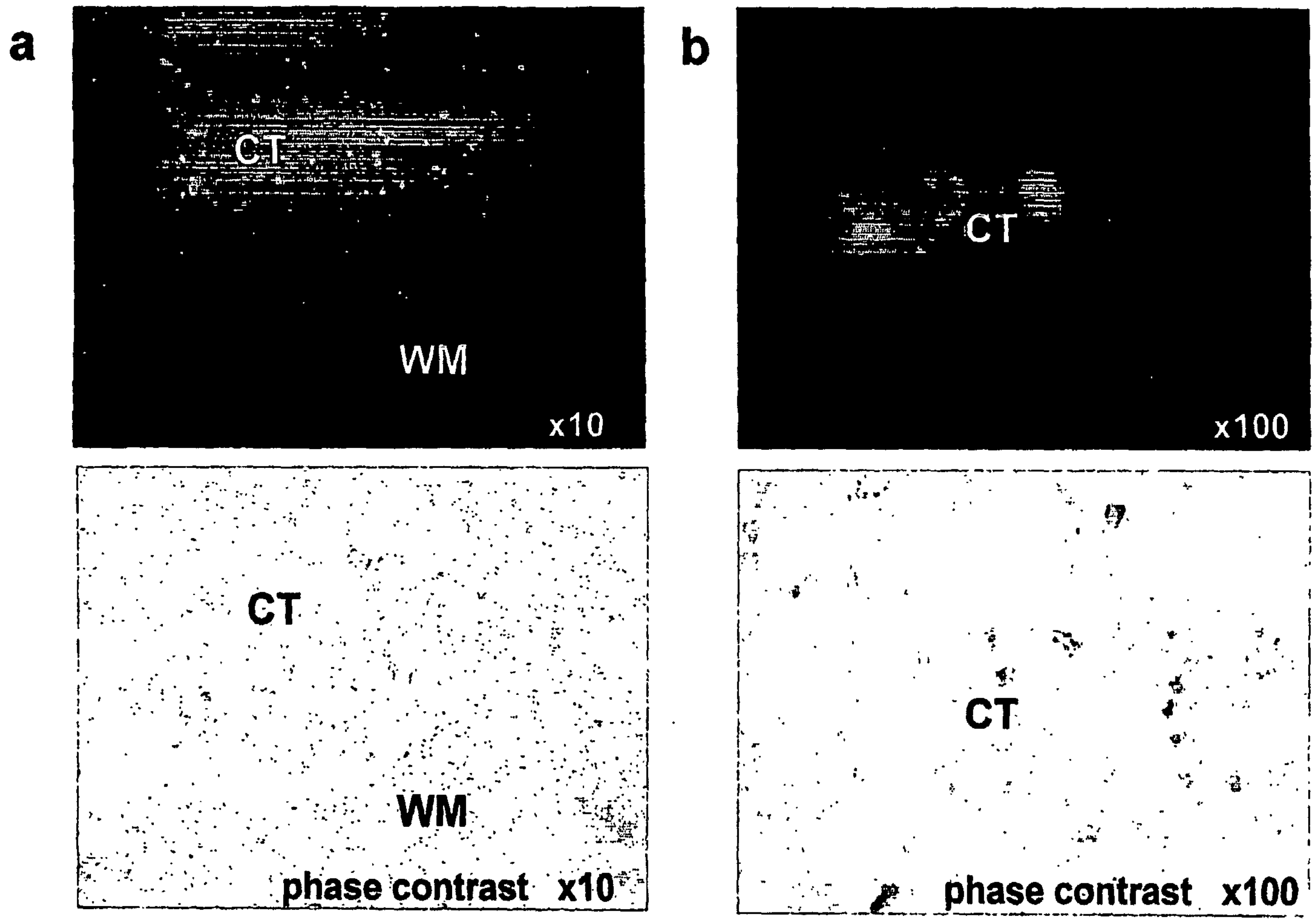

**Table 1: Differential expression of the CAPS gene in the Alzheimer's Disease brain**

| sample | Δ (fold) (frontal/ temporal cortex) |
|---|---|
| patient P012 | 3.66 |
| patient P016 | 3.02 |
| patient P010 | 4.73 |
| patient P011 | 4.11 |
| patient P014 | 2.22 |
| patient P017 | 0.90 |
| patient P019 | 3.13 |
| control C011 | 0.69 |
| control C012 | 1.15 |
| control C014 | 0.52 |
| control C005 | 1.18 |
| control C008 | 0.73 |